# EUROPEAN PATENT APPLICATION

(11) **EP 3 964 581 A1**
(43) Date of publication of application: **09.03.2022**
(21) Application number: 20799100.1
(22) Date of filing: 29.04.2020
(51) Int. Cl.: C12N 15/11, C12Q 1/6886

(54) **TUMOR MARKER STAMP-EP7 BASED ON METHYLATED MODIFICATION AND USE THEREOF**

(30) Priority: 30.04.2019 CN 201910363081
(71) Applicant: Shanghai Epiprobe Biotechnology Co., Ltd., Shanghai 200233 (CN)
(72) Inventor: LI, Zhenyan, Shanghai 200233 (CN); DONG, Shihua, Shanghai 200233 (CN)
(74) Representative: Office Kirkpatrick
(86) International application number: PCT/CN2020/087883
(87) International publication number: WO 2020/221314

(57) **Abstract**

Provided are an isolated polynucleotide and a use thereof, wherein the polynucleotide is a polynucleotide as shown in SEQ ID NO:1, a fragment with a modified CpG site, or a complementary nucleic acid. Also provided are the use of the polynucleotide for preparing a tumor detection reagent or a kit, a preparation method therefor, a reagent and a use thereof, a kit and a method for detecting the methylation profile of samples in vitro.

## Description

### Technical field

The disclosure is in the field of markers for diagnosing diseases. More specifically, the disclosure relates to a methylation based tumor marker STAMP, Specific Tumor Aligned Methylation of Pan-cancer, and use thereof.

### BACKGROUND OF DISCLOSURE

Tumors have been considered a genetic disease for decades. Several large-scale systematic sequencings for human have confirmed that the number of somatic mutations in cancer tissues is significantly less than expected. These results suggest that cancer is not a simple genetic disease.

In order to diagnose tumor, many new tumor markers have been discovered and used for clinical diagnosis in recent years. Before 1980, tumor markers were mainly hormones, enzymes, proteins and other cell secretions, such as carcinoembryonic antigen (CEA) and alpha fetoprotein (AFP) used as markers of gastric cancer, liver cancer and other tumors, carbohydrate antigen 125 (CA125) used as a marker of cervical cancer, and prostate specific antigen (PSA) used as a marker of prostate cancer. Although these tumor markers are still used in clinic, their sensitivity and accuracy have been difficult to meet the clinical needs.

More and more evidences show that small changes in epigenetic regulation play an important role in tumors. Epigenetics is a subject that studies that the heritable change of gene function without a change of DNA sequence, which eventually leads to the change of phenotype. Epigenetics mainly includes DNA methylation, histone modification, changes of microRNA level and other biochemical processes. DNA methylation is one of the epigenetic mechanisms, refers to the process of transferring methyl from S-adenosylmethionine (methyl donor) to specific bases under the catalysis of DNA methyltransferase. However, DNA methylation in mammals mainly occurs at the C of 5'-CpG-3', which results in 5-methylcytosine (5mC).

Fluid biopsy is a technique for the diagnosis and prediction of tumors using circulating tumor cells or circulating tumor DNA as detection targets. The technology has many shortcomings. First, the sensitivity and specificity are not good enough. The tumor itself is heterogeneous, including a variety of subtypes of cell populations. The proportion of tumor DNA in clinical samples, especially blood samples, is very low. The existing tumor markers are difficult to meet the sensitivity of clinical requirements, and it is easy to cause misdiagnosis. Second, one marker has good effect only for one or a few kinds of tumors. As the DNA sources in blood are very complex, the existing tumor markers cannot solve the complex problems of tumor source and metastasis. Because of these complexities, it is difficult for many DNA methylation tumor markers to have a unified standard in clinical application, which seriously affects the sensitivity and accuracy of the markers.

In the previous study of the inventor, some tumor markers based on methylation modification were found. However, to provide more ways for tumor diagnosis, it is still necessary to find more new tumor markers.

### SUMMARY OF DISCLOSURE

The object of the disclosure is to provide a method for detecting tumor based on abnormal hypermethylation of specific sites in tumor using DNA methylation modification as a tumor marker.

The first aspect of the present disclosure provides an isolated polynucleotide, including: (a) a polynucleotide with a nucleotide sequence as shown in SEQ ID NO: 1; (b) a fragment of the polynucleotide of (a), having at least one (such as 2-77, more specifically 3, 5, 10, 15, 20, 25, 30, 40, 50, 60, 70) modified CpG site; and/or (c) a nucleic acid (such as the polynucleotide with a nucleotide sequence as shown in SEQ ID No: 3) complementary to the polynucleotide or fragment of (a) or (b).

In a preferable embodiment, the modification includes 5-methylation(5mC), 5-hydroxymethylation(5hmC), 5-formylcytosine(5fC) or 5-carboxylcytosine(5-caC).

In a preferable embodiment, in (b), the fragment of the polynucleotide contains bases at positions 281 to 309 (including methylation sites 017 to 020) or positions 282 to 308 (including methylation sites 017 to 020) of SEQ ID NO: 1;
positions 1 to 389 (including methylation sites 001 to 025) of SEQ ID NO: 1; or
positions 418 to 970 (including methylation sites 026 to 077) of SEQ ID NO: 1.

The second aspect of the disclosure provides an isolated polynucleotide, which is converted from the polynucleotide, and as compared with the sequence of the first aspect, its cytosine C of the CpG site(s) with modification is unchanged, and the unmodified cytosine is converted into T or U.

In a preferable embodiment, it is converted from the polynucleotide corresponding to the first aspect by bisulfite treatment. In another preferable embodiment, the polynucleotide includes: (d) a polynucleotide with a nucleotide sequence as shown in SEQ ID NO: 2 or 4; (b) a fragment of the polynucleotide of (d), having at least one (such as 2-77, more specifically 3, 5, 10, 15, 20, 25, 30, 40, 50, 60, 70) CpGs site with modification.

In a preferable embodiment, in (e), the fragment of the polynucleotide contains bases at positions 281 to 309 (including methylation sites 017 to 020) or positions 282 to 308 (including methylation sites 017 to 020) of SEQ ID NO: 2; positions 1 to 389 (including methylation sites 001 to 025) of SEQ ID NO: 2; positions 418 to 970 (including methylation sites 026 to 077) of SEQ ID NO: 2. positions 662 to 690 (corresponding to methylation sites 017 to 020) or positions 663 to 689 (corresponding to methylation sites 017 to 020) of SEQ ID NO: 4; positions 582 to 970 (corresponding to methylation sites 001 to 025) of SEQ ID NO: 4; positions 1 to 553 (corresponding to methylation sites 026 to 077) of SEQ ID NO: 4.

The third aspect of the disclosure provides a use of the polynucleotide described in the first or second aspect in manufacture of a tumor detection agent or kit.

In a preferable embodiment, the tumors include (but are not limited to): hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; digestive system tumors such as esophageal cancer (cancer of the esophagus), gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

In another preferable embodiment, samples of the tumor include but are not limited to: tissue samples, paraffin embedded samples, blood samples, pleural effusion samples, and alveolar lavage fluid samples, ascites and lavage fluid samples, bile samples, stool samples, urine samples, saliva samples, sputum samples, cerebrospinal fluid samples, cell smear samples, cervical scraping or brushing samples, tissue and cell biopsy samples.

The fourth aspect of the disclosure provides a method of preparing a tumor detection agent, including: providing the polynucleotide described in the first or second aspect, designing a detection agent for specifically detecting the modification on CPG site(s) of a target sequence which is the full length or fragment of the polynucleotide; wherein, the target sequence has at least one (such as 2-77, more specifically, 3, 5, 10, 15, 20, 25, 30, 40, 50, 60, 70) modified CpG site; preferably, the detection agent includes (but is not limited to) primers or probes.

The fifth aspect of the disclosure provides an agent or a combination of agents which specifically detect the modification on CPG site(s) of a target sequence, which is the full length or fragment of any of the polynucleotides described in the first or second aspect and has at least one (such as 2-77, more specifically, 3, 5, 10, 15, 20, 25, 30, 40, 50, 60, 70) modified CpG site.

In a preferable embodiment, the agent or combination of agents is for a gene sequence containing the target sequence (designed based on the gene sequence), and the gene sequence includes gene Panels or gene groups.

In another preferable embodiment, the detection agent comprises: primers or probes.

In another preferable embodiment, the primers are: the primers shown in SEQ ID NO: 3 and 4; the primers shown in SEQ ID NO: 7 and 8, or the primers shown in SEQ ID NO: 9 and 10.

In the sixth aspect of the disclosure, a use of the agent or combination of agents described in the fifth aspect of the disclosure in the manufacture of a kit for detecting tumors is provided; preferably, the tumors include (but are not limited to): hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; digestive system tumors such as esophageal cancer (cancer of the esophagus), gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

The seventh aspect of the present disclosure provides a detection kit, comprising container(s) and the agent or combination of agents described above in the container(s); preferably, each agent is located in an independent container.

In a preferable embodiment, the kit also includes: bisulfite, DNA purification agent, DNA extraction agent, PCR amplification agent and/or instruction for use (indicating operation steps of the detection and a result judgment standard).

In the eighth aspect of the disclosure, a method for detecting the methylation profile of a sample *in vitro* is provided, including: (i) providing the sample and extracting the nucleic acid; (ii) detecting the modification on CPG site(s) of a target sequence in the nucleic acid of (i), wherein the target sequence is the polynucleotide described in the first aspect or the polynucleotide converted therefrom as described in the second aspect.

In a preferable embodiment, in step (3), the analysis methods include pyrosequencing, bisulfite conversion sequencing, method using methylation chip, qPCR, digital PCR, second generation sequencing, third generation sequencing, whole genome methylation sequencing, DNA enrichment detection, simplified bisulfite sequencing technology, HPLC, MassArray, methylation specific PCR (MSP), or their combination, as well as *in vitro* detection and *in vivo* tracer detection for the combined gene group of partial or all of the methylation sites in the sequence shown in SEQ ID NO: 1. In addition, other methylation detection methods and newly developed methylation detection methods in the future can be applied to the disclosure.

In another preferable embodiment, step (ii) includes: (1) treating the product of (i) to convert the unmodified cytosine into uracil; preferably, the modification includes 5-methylation, 5-hydroxymethylation, 5-formylcytosine or 5- carboxylcytosine; preferably, treating the nucleic acid of step (i) with bisulfite; and (2) analyzing the modification of the target sequence in the nucleic acid treated by (1).

In another preferable embodiment, the abnormal methylation profile is the high level of methylation of C in CPG(s) of the polynucleotide.

In another preferable embodiment, the methylation profile detecting method is not for the purpose of directly obtaining the diagnosis result of a disease, or is not a diagnostic method.

The ninth aspect of the disclosure provides a tumor diagnosis kit, including a primer pair designed based on the sequence described in the first or second aspect of the disclosure, and gene Panels or gene groups containing the sequence, to obtain the characteristics of normal cells and tumor cells through DNA methylation detection.

Other aspects of the disclosure will be apparent to those skilled in the art based on the disclosure herein.

### BRIEF DESCRIPTION OF DRAWINGS

Figure 1, BSP assay for methylation levels of the methylation sites 001 to 025 in SEQ ID NO:1 region in lung cancer cells and normal lung cells. Dark squares indicate "methylated".
Figure 2, BSP assay for methylation levels of the methylation sites 026 to 077 in SEQ ID NO:1 region in lung cancer cells and normal lung cells. Dark squares indicate "methylated".
Figure 3, comparison of methylation values of STAMP-EP7 between non-cancer tissues and cancer tissues for clinical samples of leukemia.
Figure 4, comparison of methylation values of STAMP-EP7 between paracancerous samples and cancer tissue samples for clinical samples of breast cancer.
Figure 5, comparison of methylation values of STAMP-EP7 between paracancerous samples and cancer tissue samples for clinical samples of rectal cancer.
Figure 6, comparison of methylation values of STAMP-EP7 between paracancerous samples and cancer tissue samples for clinical samples of esophageal cancer.
Figure 7, comparison of methylation values of STAMP-EP7 between paracancerous samples and cancer tissue samples for clinical samples of liver cancer.
Figure 8, comparison of methylation values of STAMP-EP7 between paracancerous samples and cancer tissue samples for clinical samples of lung cancer.
Figure 9, comparison of methylation values of STAMP-EP7 between paracancerous samples and cancer tissue samples for clinical samples of pancreatic cancer.

### DETAILED DESCRIPTION

The inventor is committed to the research of tumor markers. After extensive research and screening, the inventor provides a universal DNA methylation tumor marker, STAMP (Specific Tumor Aligned Methylation of Pan-cancer). In normal tissues, STAMP was hypomethylated, while in tumor tissues, it was hypermethylated. It can be used for clinical tumor detection and as the basis of designing tumor diagnostic agents.

### Terms

As used herein, "isolated" refers to a material separated from its original environment (if the material is a natural material, the original environment is the natural environment). For example, in living cells, polynucleotides and polypeptides in their natural state are not isolated or purified, but the same polynucleotides or polypeptides will be isolated ones if they are separated from other substances existed in the natural state.

As used herein, "sample" includes substances suitable for DNA methylation detection obtained from any individual or isolated tissue, cell or body fluid (such as plasma). For example, the samples include but are not limited to: tissue samples, paraffin embedded samples, blood samples, pleural effusion samples, and alveolar lavage fluid samples, ascites and lavage fluid samples, bile samples, stool samples, urine samples, saliva samples, cerebrospinal fluid samples, cell smear samples, cervical scraping or brushing samples, tissue and cell biopsy samples.

As used herein, "hypermethylation" refers to high level of methylation, hydroxymethylation, formylcytosine or carboxylcytosine of CpG in a gene sequence. For example, in the case of methylation specific PCR (MSP), if the PCR reaction with methylation specific primers has positive PCR results, the DNA (gene) region of interest is in hypermethylation state. For another example, in the case of real-time quantitative methylation specific PCR, hypermethylation can be determined based on statistic difference of the methylation status value as compared with the control sample.

### Gene marker

In order to find a useful target for tumor diagnosis, the inventor has identified the target of STAMP-EP7 after extensive and in-depth research. The methylation status of the sequence of STAMP-EP7 gene is significantly different between tumor tissues and non-tumor tissues. If the abnormal hypermethylation of the sequence of STAMP-EP7 gene of a subject is detected, the subject can be identified as having a high-risk of tumor. Moreover, the significant difference of STAMP-EP7 between tumor and non-tumor tissues exists in different kinds of tumors, including solid tumors and non-solid tumors.

Therefore, the disclosure provides an isolated polynucleotide, comprising the nucleotide sequence shown in the sequence of SEQ ID NO: 1 or SEQ ID NO: 3 (the reverse complementary sequence of SEQ ID NO: 1). For tumor cells, the polynucleotide contains 5-methylcytosine (5mC) at C positions of many 5'-CpG-3'. The disclosure also comprises a fragment of the polynucleotide with a nucleotide sequence as shown in SEQ ID NO: 1 or 3, having at least one (such as 2-77, more specifically 3, 5, 10, 15, 20, 25, 30, 40, 50, 60, 70) methylated CpG site. The above polynucleotides or fragments can also be used in the design of detection agents or detection kits.

In some specific embodiments of the disclosure, the fragment of the polynucleotide is, for example, a fragment containing the residues 281-309 of SEQ ID NO: 1 (containing CpG sites 017-020). Antisense strands of the above fragments are suitable for use in the disclosure. These fragments are merely examples of preferable embodiments of the present disclosure. Based on the information provided by the present disclosure, other fragments can also be selected.

In addition, gene Panels or gene groups containing the sequence shown in the SEQ ID NO: 1 or SEQ ID NO: 2 or a fragment thereof are also encompassed by the disclosure. For the gene Panel or gene group, the characteristics of normal cells and tumor cells can also be identified through DNA methylation detection.

The above polynucleotides can be used as the key regions for analysis of the methylation status in the genome. Their methylation status can be analyzed by various technologies known in the art. Any technique that can be used to analyze the methylation state can be applied to the present disclosure.

When treated with bisulfite, un-methylated cytosine(s) of the above polynucleotides will be converted into uracil, while methylated cytosine(s) remained unchanged.

Therefore, the disclosure also provides the polynucleotides obtained from the above polynucleotides (including the complementary chain (antisense chain)) after being treated with bisulfite, including the polynucleotides with a nucleotide sequence as shown in SEQ ID NO: 2 or SEQ ID NO: 4. These polynucleotides can also be used in the design of detection agents or detection kits.

The disclosure also comprises a fragment of the polynucleotides obtained from the above polynucleotides or the antisense strand thereof after being treated with bisulfite, wherein the fragment contains at least one (such as 2-77, more specifically 3, 5, 10, 15, 20, 25, 30, 40, 50, 60, 70) methylated CpG site. The No. of each CpG site in the antisense strand corresponding to the number of the sense strand is readily obtained according to the content described by the present disclosure.

### Detection agents and kits

Based on the new discovery of the disclosure, a detection agent designed based on said polynucleotide(s) is also provided for detecting the methylation profile of polynucleotide(s) in the sample *in vitro.* The detection methods and agents known in the art for determining the sequence, variation and methylation of the genome can be applied in the disclosure.

Therefore, the disclosure provides a method of preparing a tumor detection agent, including: providing the polynucleotide, designing a detection agent for specifically detecting a target sequence which is the full length or fragment of the polynucleotide; wherein, the target sequence has at least one methylated CpG site.

The detection agent herein includes but is not limited to: primers or probes, etc.

For example, the agent is a primer pair. Based on the sequence of the polynucleotide, those skilled in the art know how to design primer(s). The two primers are on each side of the specific sequence of the target gene to be amplified (including CpG sequence, for the gene region originally methylated, the primer is complementary with CpG, and for the gene region originally un-methylated, the primer is complementary with TpG). It should be understood that based on the new discovery of the disclosure, those skilled in the art can design a variety of primers or probes or other types of detection agents for CpG sites at different positions on the target sequence or their combinations. These primers or probes or other types of detection agents should be included in the technical solution of the disclosure. In a preferable example of the disclosure, the primers are: the primers shown in SEQ ID NO: 5 and 6, which could be used to amplify a product containing CpG sites 001-025 of SEQ ID NO: 1; or, the primers shown in SEQ ID NO: 7 and 8, which could be used to amplify a product containing CpG sites 026-077 of SEQ ID NO: 1; or the primers shown in SEQ ID NO: 9 and 10, which could be used to amplify a product containing CpG sites 017-020 of SEQ ID NO: 1.

The agent can also be a combination of agents (primer combination), including more than one set of primers, so that the multiple polynucleotides can be amplified respectively.

The disclosure also provides a kit for detecting the methylation profile of polynucleotide in a sample *in vitro,* which comprises container(s) and the above primer pair(s) in the container(s).

In addition, the kit can also include various reagents required for DNA extraction, DNA purification, PCR amplification, etc.

In addition, the kit can also include an instruction for use, which indicates operation steps of the detection and a result judgment standard, for the application of those skilled in the art.

### Detection method

The methylation profile of a polynucleotide can be determined by any technique in the art (such as methylation specific PCR (MSP) or real-time quantitative methylation specific PCR, Methylight), or other techniques that are still developing and will be developed.

Quantitative methylation specific PCR (QMSP) can also be used to detect methylation level. It is a continuous optical monitoring method based on fluorescent PCR, which is more sensitive than MSP. It has high throughput and avoids electrophoresis based result analysis.

Other available technologies include conventional methods in the art such as pyrosequencing, bisulfite converstion sequencing, qPCR, second generation sequencing, whole genome methylation sequencing, DNA enrichment detection, simplified bisulfite sequencing or HPLC, and combined gene group detection. It should be understood that, on the basis of the new disclosure herein, these well-known technologies and some technologies to be developed in the art can be applied to the present disclosure.

As a preferable embodiment of the disclosure, a method of detecting the methylation profile of a polynucleotide in a sample *in vitro* is also provided. The method is based on the follow principle: the un-methylated cytosine can be converted into uracil by bisulfite, which can be transformed into thymine in the subsequent PCR amplification process, while the methylated cytosine remains unchanged; therefore, after the polynucleotide is treated by bisulfite, the methylated site presents a polynucleotide polymorphism (SNP) similar to C/T. Based on the above principle, methylated and un-methylated cytosine can be distinguished by identifying the methylation profile of a polynucleotide in the sample.

The method of the disclosure includes: (a) providing samples and extracting genomic DNA; (b) treating the genomic DNA of step (a) with bisulfite, so as to convert the un-methylated cytosine in the genomic DNA into uracil; (c) analyzing whether the genomic DNA treated in step (b) contains an abnormal methylation profile.

The method of the disclosure can be used for: (i) analyzing whether a subject has tumor by detecting the sample of the subject; (ii) identifying a population having high-risk of tumor. The method needs not to be aimed at obtaining direct diagnosis results.

In a preferable embodiment of the disclosure, DNA methylation is detected by PCR amplification and pyrosequencing. It should be understood by those in the art that DNA methylation detection is not limited to these methods, and any other DNA methylation detection method can be used. The primers used in PCR amplification are not limited to those provided in Examples.

Because of bisulfite treatment, in which un-methylated cytosine in genomic DNA are converted into uracil and then transformed into thymine in the subsequent PCR process, the sequence complexity of the genome will be reduced, and it will be more difficult to amplify specific target fragments by PCR. Therefore, in order to improve amplification efficiency and specificity, nested PCR may be preferable, wherein two sets of primers (outer primers and inner primers) are used in two successive runs of PCR, and the product from the first run undergoes a second run with the second set of primers. However, it should be understood that the detection methods suitable for the present disclosure are not limited thereto.

After the research and verification on clinical samples, the method of the disclosure provides very high accuracy in the clinical diagnosis of tumors. The disclosure can be applied to the fields of tumor auxiliary diagnosis, efficacy evaluation, prognosis monitoring, etc., thus has a high clinical value.

The disclosure is further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### Example 1. Nucleic acid sequence for STAMP-EP7 detection

Based on extensive research and screening, a tumor marker STAMP-EP7 is provided and its sequence is as follows: SEQ ID NO: 1 (chr14:609S1977-609S2946, Human/hg19), in which the underlined bases are methylated CpG sites, and each number below the underline indicates the site number.

CGGGCCAAGCCCAACGAGCGCTTCAGAAATCGTTGTTGAGTCGGGGCACGACTTGAAG
01 02 03 04 05 06
TTTTGGGGATGGGGTGGGAAGCACCCGCATAACCGCTTATGCGTGGGATTGCAACAAG
07 08 09
CATCCGGATCTTCCAGGGTTCACGGACACCCAGATTTCTCCACAGGATATGCCCTTTAA
10 11
AGTAAACGCTCTGAATATGTCCCTTGGTGGCGTGTGTACAACCACGTGTAGCAAAATTC
12 13 14
TAACGAAAGCCCTTCCCCAACACCCGGGTAAACCTAGAGAAACAGTGCGATTTCGCATT
15 16 17 18
TACGAATTTACCACGAAGAATAAAAAAATTGGAGGTTTTCACAGAACAGCCAATTCGC
19 20 21
ATTACGGCATACGCCTGAGGCGGGGTTCCTGTCTACCGTGCCCCTCAGAGCCAGGAAC
22 23 24 25
CACAGCCCGTGCGCCTCCCGCAGTGGGAGTTCGCCGGCCGACTCCCACCCTCACAGCCT
26 27 28 29 30 31
CCTGTCCTGGCTTCCCCTCGCCCGAGGCTGCAACACCGCATCCCCCCCATCCCCCGCCG
32 33 34 35 36
CGCCCTCAGCCTCGGGCCGCACCAACCCAGGGGATAAGGCGACTCCGGTCGCTCTGAG
37 38 39 40 41 42
GGGCAGGGCCAGCCAGCCCCCTCCCACCCACGCACACGCTCCCCCTCAGAGCCGCCGG
43 44 45 46
CCCAGAGAAAAACCGCCACATGCAGCTCCCTTCCACACGCACCTAAACAGCTCCTCTGG
47 48
ACCCGAACGCCCACACCCTCCCTCCCTGGGGTCCCAAACTCCACTCAGGACGCCACAG
49 50 51
CGGATCCTAACTACAAACGGTCCCCGGAGCCCTGGGCTGGACTCGCTCAGCCCCGCCCC
52 53 54 55 56
CACGCCCCTGGTACCAGCCCTGAGAGACCCCGCGGAGCACGCCGCGGGAGCCGCAGAT
57 58 59 60 61 62 63
CGCGCTGAAGAGCAGCGAGATCGCGCTCTGGACGAGACCTGCGCGGCTGCAACCGCTC
64 65 66 67 68 69 70 71 72
CTTCTTCGCGGGTGGAAGCGCGGAATAAGCCCCG
73 74 75 76 77

The bisulfite treated sequence of SEQ ID NO: 1 is shown in SEQ ID NO: 2 (Y represents C or U) as follows:

YGGGUUAAGUUUAAYGAGYGUTTUAGAAATYGTTGTTGAGTYGGGGUA
1                    2    3                4              5
YGAUTTGAAGTTTTGGGGATGGGGTGGAAGUAUUYGUATAAUYGUTTA
6                                          7          8
TGYGTGGGATTGUAAUAAGUATUYGGATUTTUAGGGTTUAYGGAUAUU
   9                    10                    11
UAGATTTUTUUAUAGGATATGUUUTTTAAAGTAAAYGUTUTGAATATGT
                                    12
UUUTTGGTGGYGTGTGTAUAAUUAYGTGTAGUAAAATTUTAAYGAAAGU
           13             14                  15
UUTTUUUUAAUAUUYGGGTAAAUUTAGAGAAAUAGTGYGATTTYGUATT
               16                      17        18
TAYGAATTTAUUAYGAAGAATAAAAAAATTGGAGGTTTUAUAGAAUAG
  19           20
UUAATTYGUATTAYGGUATAYGUUTGAGGYGGGGTTUUTGTUTAUYGTG
       21        22       23        24                25
UUUUTUAGAGUUUAGGAAUUAUAGUUYGTGYGUUTUUYGUAGTGGGAG
                           26   27      28
TTYGUYGGUYGAUTUUUAUUUTUAUAGUUTUUTGUUTGGUTTUUUUT
  29  30   31
YGUUYGAGGUTGUAAUAUYGUATUUUUUUUATUUUUYGUYGYGUUUTU
32   33             34                  35  36 37
AGUUTYGGGUYGUAUUAAUUUAGGGGATAAGGYGAUTUYGGTYGUTUT
      38    39                    40     41  42
GAGGGGUAGGGUUAGUUAGUUUUUTUUUAUUUAYGUAUAYGUTUUUUU
                                  43     44
TUAGAGUYGUYGGUUUAGAGAAAAAUYGUUAUATGUAGUTUUUTTUUA
        45  46              47
UAYGUAUUTAAAUAGUTUUTUTGGAUUYGAAYGUUUAUAUUUTUUUTU
  48                         49   50
UUTGGGGTUUUAAAUTUUAUTUAGGAYGUUAUAGYGGATUUTAAUTAU
                           51      52
AAAYGGTUUUYGGAGUUUTGGGUTGGAUTYGUTUAGUUUYGUUUUUA
   53      54                 55         56
YGUUUUTGGTAUUAGUUUTGAGAGAUUUYGYGGAGUAYGUYGYGGGAG
57                             58  59     60  61 62
UYGUAGATYGYGUTGAAGAGUAGYGAGATYGYGUTUTGGAYGAGAUUT
 63      64  65           66     67  68      69
GYGYGGUTGUAAUYGUTUUTTUTTYGYGGGTGGAAGYGYGGAATAAGU
 70  71         72          73  74        75  76
UUYG
  77

The reverse complementary sequence of SEQ ID NO: 1 is shown in SEQ ID NO: 3 as follows:

CGGGGCTTATTCCGCGCTTCCACCCGCGAAGAAGGAGCGGTTGCAGCCGCGCAGGTCTCGTCC
AGAGCGCGATCTCGCTGCTCTTCAGCGCGATCTGCGGCTCCCGCGGCGTGCTCCGCGGGGTCTC
TCAGGGCTGGTACCAGGGGCGTGGGGGCGGGGCTGAGCGAGTCCAGCCCAGGGCTCCGGGGA
CCGTTTGTAGTTAGGATCCGCTGTGGCGTCCTGAGTGGAGTTTGGGACCCCAGGGAGGGAGGG
TGTGGGCGTTCGGGTCCAGAGGAGCTGTTTAGGTGCGTGTGGAAGGGAGCTGCATGTGGCGGT
TTTTCTCTGGGCCGGCGGCTCTGAGGGGGAGCGTGTGCGTGGGTGGAGGGGGCTGGCTGGCC
CTGCCCCTCAGAGCGACCGGAGTCGCCTTATCCCCTGGGTTGGTGCGGCCCGAGGCTGAGGGC
GCGGCGGGGGATGGGGGGGATGCGGTGTTGCAGCCTCGGGCGAGGGGAAGCCAGGACAGGAG
GCTGTGAGGGTGGGAGTCGGCCGGCGAACTCCCACTGCGGGAGGCGCACGGCTGTGGTTCCT
GGGCTCTGAGGGGCACGGTAGACAGGAACCCCGCCTCAGGCGTATGCCGTAATGCGAATTGGC
TGTTCTGTGAAAACCTCCAATTTTTTTATTCTTCGTGGTAAATTCGTAATGCGAAATCGCACTG
TTTCTCTAGGTTTACCCGGGTGTTGGGGAAGGGCTTTCGTTAGAATTTTGCTACACGTGGTTGTA
CACACGCCACCAAGGGACATATTCAGAGCGTTTACTTTAAAGGGCATATCCTGTGGAGAAATC
TGGGTGTCCGTGAACCCTGGAAGATCCGGATGCTTGTTGCAATCCCACGCATAAGCGGTTATGC
GGGTGCTTCCCACCCCATCCCCAAAACTTCAAGTCGTGCCCCGACTCAACAACGATTTCTGAAG
CGCTCGTTGGGCTTGGCCCG

The bisulfite treated sequence of SEQ ID NO: 3 is shown in SEQ ID NO: 4 (Y represents C or U) as follows:

YGGGGUTTATTUYGYGUTTUUAUUYGYGAAGAAGGAGYGGTTGUAGUYGYGUAGGTUTYGTU
UAGAGYGYGATUTYGUTGUTUTTUAGYGYGATUTGYGGUTUUYGYGGYGTGUTUYGYGGGGT
UTUTUAGGGUTGGTAUUAGGGGYGTGGGGGYGGGGUTGAGYGAGTUUAGUUUAGGGUTUYG
GGGAUYGTTTGTAGTTAGGATUYGUTGTGGYGTUUTGAGTGGAGTTTGGGAUUUAGGGAGG
GAGGGTGTGGGYGTTYGGGTUUAGAGGAGUTGTTTAGGTGYGTGTGGAAGGGAGUTGUATGT
GGYGGTTTTTUTUTGGGUYGGYGGUTUTGAGGGGGAGYGTGTGYGTGGGTGGGAGGGGGUTG
GUTGGUUUTGUUUUTUAGAGYGAUYGGAGTYGUUTTATUUUUTGGGTTGGTGYGGUUYGAGG
UTGAGGGYGYGGYGGGGGATGGGGGGGATGYGGTGTTGUAGUUTYGGGYGAGGGGAAGUUA
GGAUAGGAGGUTGTGAGGGTGGGAGTYGGUYGGYGAAUTUUUAUTGYGGGAGGYGUAYGGG
UTGTGGTTUUTGGGUTUTGAGGGGUAYGGTAGAUAGGAAUUUYGUUTUAGGYGTATGUYGTA
ATGYGAATTGGUTGTTUTGTGAAAAUUTUUAATTTTTTTATTUTTYGTGGTAAATTYGTAAATG
YGAAATYGUAUTGTTTUTUTAGGTTTAUUYGGGTGTTGGGGAAGGGUTTTYGTTAGAATTTG
UTAUAYGTGGTTGTAUAUAYGUUAUUAAGGGAUATATTUAGAGYGTTTAUTTTAAAGGGUAT
ATUUTGTGGAGAAATUTGGGTGTUYGTGAAUUUTGGAAGATUYGGATGUTTGTTGUAATUUU
AYGUATAAGYGGTTATGYGGGTGUTTUUUAUUUUAUUUUAAAAUTTUAAGTYGTGUUUYGA
UTUAAUAAYGATTTUTGAAGYGUTYGTTGGGUTTGGUUYG

### Example 2. Difference in methylation of STAMP-EP7 CpG sites between tumor cells and non-tumor cells -- Bisulfite Sequencing PCR (BSP)

Bisulfite Sequencing PCR comprises the following steps:
1. Genomic DNA was extracted from lung cancer cell line A549 and normal lung cell line MRC5;
2. The extracted genomic DNA of lung cancer cell line A549 and normal lung cell line MRC5 were treated with bisulfite and used as templates for subsequent PCR amplification;
3. Primers (SEQ ID NO: 5-8)were designed according to SEQ ID NO: 1 by conventional methods, and used for amplification;
4. After PCR amplification, 2% agarose gel electrophoresis was used to detect the specificity of the PCR fragments. The target fragments were recovered by gel cutting and connected to T vector which was transformed into competent *Escherichia coli.* The bacteria were spread on plate, and clones were selected the next day and sequenced. More than ten clones were selected for each fragment for Sanger sequencing.

**Table 1, BSP primer**

| Primer Name | 5'-3' Primer Sequence | Detected CpG site No. |
|---|---|---|
| STAMP-EP7-Sanger-Pri mer-F1 | TGAGTTTATGGYGATATTTTTAGTT (SEQ ID NO: 5) | CpG 001-025 of SEQ ID NO: 1 |
| ST AM P-EP7-Sanger-Pri mer-R1 | CTATAATTCCTAAACTCTAAAAAAC (SEQ ID NO: 6) | |
| STAMP-EP7-Sanger-Pri mer-F2 | GTTTTTTAGAGTTTAGGAATTATAG (SEQ ID NO: 7) | CpG 026-077 of SEQ ID NO: 1 |
| ST AM P-EP7-Sanger-Pri mer-R2 | CAACACCCACTTCT AT AATTTT AC (SEQ ID NO: 8) | |

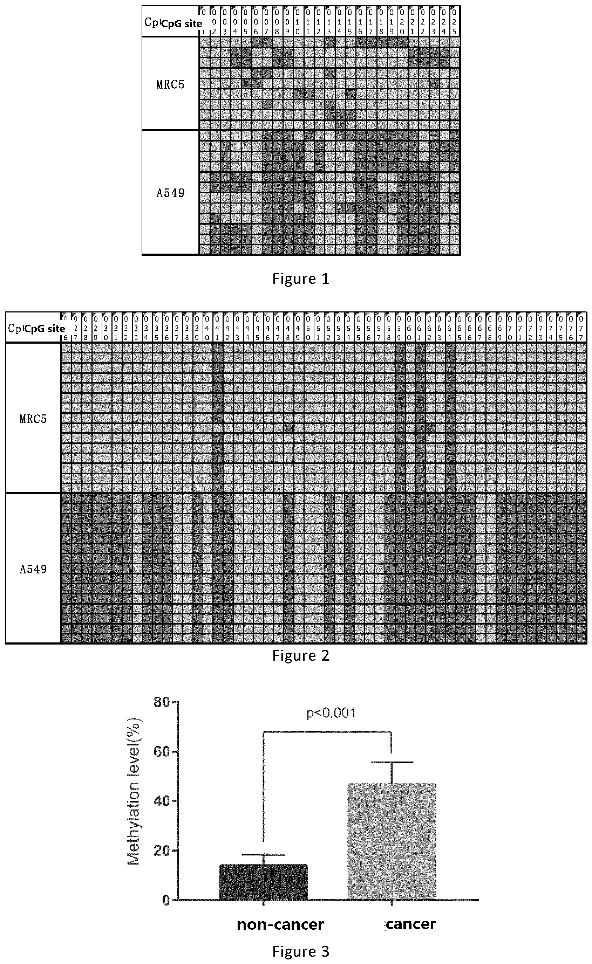

Figure 1 shows BSP assay for methylation levels of the methylation sites 001 to 025 in SEQ ID NO:1 region in lung cancer cells and normal lung cells. It is indicates that the methylation level of STAMP-EP7 of lung cancer cells is significantly higher than that of normal lung cells.

Figure 2 shows BSP assay for methylation levels of the methylation sites 026 to 077 in SEQ ID NO:1 region in lung cancer cells and normal lung cells. It is indicates that the methylation level of STAMP-EP7 of lung cancer cells is significantly higher than that of normal lung cells.

### Example 3: Difference in methylation of STAMP-EP7 CpG sites between tumor cells and non-tumor cells-pyrosequencing

Pyrosequencing comprises the following steps:
1. Obtaining of the clinical samples: paracancerous/non-cancer were used as the control group, and cancer tissue samples were used as the experimental group;
2. DNA extraction: DNA was extracted from the experimental group and the control group respectively. Phenol-chloroform extraction method was used in this experiment, which is not limited thereto;
3. Bisulfite treatment: the extracted DNA samples were treated with bisulfite and the procedures were strictly followed. EZ DNA Methylation-Gold Kit (ZYMO Research, Cat# D5006) was used in this experiment, which is not limited thereto;
4. Primer design: PCR primers and pyrosequencing primers were designed according to the characteristics of SEQ ID NO: 1 of STAMP-EP7 sequence. The methylation values of STAMP-EP7 were detected as the methylation values of CpG sites 017 to 020. The PCR primers sequences, pyrosequencing primers sequences, and the pyrosequencing detecting sequences are shown as SEQ ID NO: 9-12, and the detected sites are shown in Table 2;
5. PCR amplification and agarose gel electrophoresis: The bisulfite treated samples were used as templates for PCR amplification. The specificity of PCR amplification was identified by agarose gel electrophoresis of the amplified products;
6. Pyrosequencing: Pyro Mark Q96 ID pyrosequencing instrument (QIAGEN) was used for sequencing, and the procedures in instructions were strictly followed;
7. Calculation of STAMP-EP7 methylation value: pyrosequencing can detect the methylation value of each site in the target region, respectively, and the average methylation value of all sites were calculated as the STAMP-EP7 methylation value in the sample;
8. Results analysis: the methylation value of STAMP-EP7 was compared between the paracancerous/non-cancer control group and the cancer experimental group.

**Table 2, Pyro primer**

| Primer Name | 5'-3' sequence | Detected CpG site No. |
|---|---|---|
| STAMP-EP7-Pyroseq-Primer-F | GAAAGTTTTTTTTTAATATTYGGGTAAATTTAGAGA(SE Q ID NO: 9) | Detect CpG 017-020 of SEQ ID NO: 1 |
| STAMP-EP7-Pyroseq-Primer-R(with 5'-Biotin modification) | CTATTCTATAAAAACCTCCAATTTTTTTATTCT (SEQ ID NO: 10) | |
| STAMP-EP7-Pyroseq-Primer-S eq | TATTYGGGTAAATTTAGAGAAATAGTG (SEQ ID NO: 11) | |
| pyrosequencing detecting sequences | YGATTTYGTATTTAYGAATTTATTAYGA (SEQ ID NO: 12) | |

### Example 4. STAMP-EP7: leukemia clinical sample assay - pyrosequencing

STAMP-EP7 methylation value between the control group of 8 non-leukemia bone marrow smear clinical samples and the experimental group of 8 leukemia bone marrow smear clinical samples was compared according to the pyrosequencing in Example 3.

The result in Figure 3 shows that, in clinical samples of leukemia, the methylation value of STAMP-EP7 in the experimental group was significantly higher than that of non-cancer tissues.

### Example 5. STAMP-EP7: breast cancer clinical sample assay - pyrosequencing

STAMP-EP7 methylation value between the control group of 5 cases of breast cancer paracancerous clinical samples and the experimental group of 5 cases of breast cancer clinical samples was compared according to the pyrosequencing in Example 3.

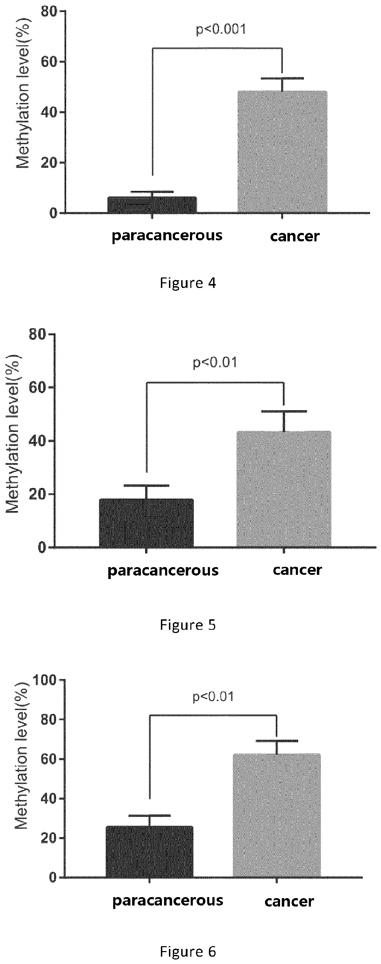

The result in Figure 4 shows that, in clinical samples of breast cancer, the methylation value of STAMP-EP7 in the experimental group was significantly higher than that of paracancerous tissues.

### Example 6. STAMP-EP7: rectal cancer clinical sample assay - pyrosequencing

STAMP-EP7 methylation value was analyzed on the control group of 8 cases of colorectal cancer paracancerous clinical samples and the experimental group of 8 cases of colorectal cancer clinical samples according to the pyrosequencing in Example 3.

The result in Figure 5 shows that, in clinical samples of colorectal cancer, the methylation value of STAMP-EP7 in the experimental group was significantly higher than that of paracancerous tissues.

### Example 7. STAMP-EP7: Esophageal cancer clinical sample assay - pyrosequencing

STAMP-EP7 methylation value was analyzed on the control group of 10 cases of esophageal cancer paracancerous clinical samples and the experimental group of 10 cases of esophageal cancer clinical samples according to the pyrosequencing in Example 3.

The result in Figure 6 shows that, in clinical samples of esophageal cancer, the methylation value of STAMP-EP7 in the experimental group was significantly higher than that of paracancerous tissues.

### Example 8. STAMP-EP7: liver cancer clinical sample assay - pyrosequencing

STAMP-EP7 methylation value was analyzed on the control group of 8 cases of liver cancer paracancerous clinical samples and the experimental group of 8 cases of liver cancer clinical samples according to the pyrosequencing in Example 3.

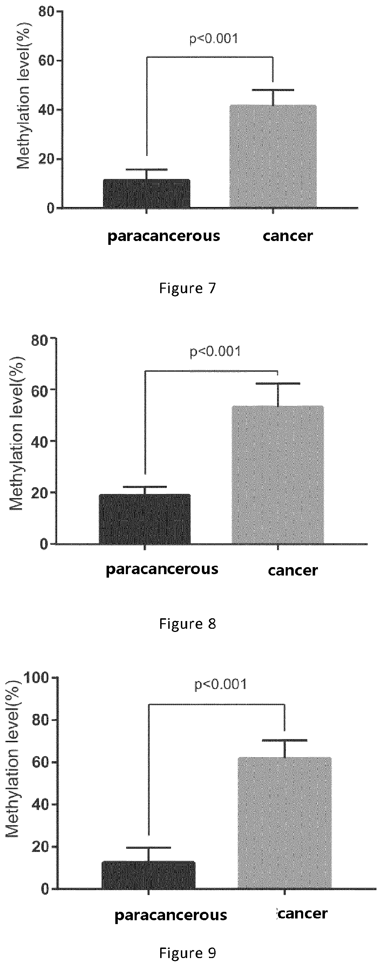

The result in Figure 7 shows that, in clinical samples of liver cancer, the methylation value of STAMP-EP7 in the experimental group was significantly higher than that of paracancerous tissues.

### Example 9. STAMP-EP7: lung cancer clinical sample assay - pyrosequencing

STAMP-EP7 methylation value was analyzed on the control group of 4 cases of lung cancer paracancerous clinical samples and the experimental group of 4 cases of lung cancer clinical samples according to the pyrosequencing in Example 3.

The result in Figure 8 shows that, in clinical samples of lung cancer, the methylation value of STAMP-EP7 in the experimental group was significantly higher than that of paracancerous tissues.

### Example 10. STAMP-EP7: pancreatic cancer clinical sample assay - pyrosequencing

STAMP-EP7 methylation value was analyzed on the control group of 4 cases of pancreatic cancer paracancerous clinical samples and the experimental group of 4 cases of pancreatic cancer clinical samples according to the pyrosequencing in Example 3.

The result in Figure 9 shows that, in clinical samples of pancreatic cancer, the methylation value of STAMP-EP7 in the experimental group was significantly higher than that of paracancerous tissues.

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

Sequence Listing

<110>  SHANGHAI EPIPROBE BIOTECHNOLOGY CO.,LTD.

<120>  TUMOR MARKER STAMP-EP7 BASED ON METHYLATION MODIFICATION AND USE
THEREOF

<130>  192646PCWO

<150>  201910363081.4
<151>  2020-04-30

<160>  12

<170>  SIPOSequenceListing 1.0

<210>  1
<211>  970
<212>  DNA
<213>  Homo sapiens

<400>  1
cgggccaagc ccaacgagcg cttcagaaat cgttgttgag tcggggcacg acttgaagtt      60
ttggggatgg ggtgggaagc acccgcataa ccgcttatgc gtgggattgc aacaagcatc     120
cggatcttcc agggttcacg gacacccaga tttctccaca ggatatgccc tttaaagtaa     180
acgctctgaa tatgtccctt ggtggcgtgt gtacaaccac gtgtagcaaa attctaacga     240
aagcccttcc ccaacacccg ggtaaaccta gagaaacagt gcgatttcgc atttacgaat     300
ttaccacgaa gaataaaaaa attggaggtt ttcacagaac agccaattcg cattacggca     360
tacgcctgag gcggggttcc tgtctaccgt gcccctcaga gcccaggaac cacagcccgt     420
gcgcctcccg cagtgggagt tcgccggccg actcccaccc tcacagcctc ctgtcctggc     480
ttcccctcgc ccgaggctgc aacaccgcat cccccccatc ccccgccgcg ccctcagcct     540
cgggccgcac caacccaggg gataaggcga ctccggtcgc tctgaggggc agggccagcc     600
agccccctcc cacccacgca cacgctcccc ctcagagccg ccggcccaga gaaaaaccgc     660
cacatgcagc tcccttccac acgcacctaa acagctcctc tggacccgaa cgcccacacc     720
ctccctccct ggggtcccaa actccactca ggacgccaca gcggatccta actacaaacg     780
gtccccggag ccctgggctg gactcgctca gccccgcccc cacgcccctg gtaccagccc     840
tgagagaccc cgcggagcac gccgcgggag ccgcagatcg cgctgaagag cagcgagatc     900
gcgctctgga cgagacctgc gcggctgcaa ccgctccttc ttcgcgggtg gaagcgcgga     960
ataagccccg                                                            970

<210>  2
<211>  970
<212>  DNA/RNA
<213>  Artificial Sequence

<220>
<221>  misc_feature
<222>  (1)..(970)
<223>  y is c or u


<400>  2
yggguuaagu uuaaygagyg uttuagaaat ygttgttgag tygggguayg auttgaagtt      60
ttggggatgg ggtgggaagu auuyguataa uyguttatgy gtgggattgu aauaaguatu     120
yggatuttuu agggttuayg gauauuuaga tttutuuaua ggatatguuu tttaaagtaa     180
aygututgaa tatgtuuutt ggtggygtgt gtauaauuay gtgtaguaaa attutaayga     240
aaguuuttuu uuaauauuyg ggtaaauuta gagaaauagt gygatttygu atttaygaat     300
ttauuaygaa gaataaaaaa attggaggtt ttuauagaau aguuaattyg uattayggua     360
tayguutgag gyggggttuu tgtutauygt guuuutuaga guuuaggaau uauaguuygt     420
gyguutuuyg uagtgggagt tyguygguyg autuuuauuu tuauaguutu utgtuutggu     480
ttuuuutygu uygaggutgu aauauyguat uuuuuuuatu uuuyguygyg uuutuaguut     540
yggguyguau uaauuuaggg gataaggyga utuyggtygu tutgaggggu aggguuaguu     600
aguuuuutuu uauuuaygua uaygutuuuu utuagaguyg uygguuuaga gaaaaauygu     660

uauatguagu tuuuttuuau ayguauutaa auagutuutu tggauuygaa yguuuauauu 720
utuuutuuut ggggtuuuaa autuuautua ggayguuaua gyggatuuta autauaaayg 780
gtuuuyggag uuutgggutg gautygutua guuuyguuuu uayguuuutg gtauuaguuu 840
tgagagauuu ygyggaguay guygygggag uyguagatyg ygutgaagag uagygagaty 900
gygututgga ygagauutgy gyggutguaa uygutuuttu ttygygggtg gaagygygga 960
ataaguuuyg 970

<210> 3
<211> 970
<212> DNA
<213> Homo sapiens

<400> 3
cggggcttat tccgcgcttc cacccgcgaa gaaggagcgg ttgcagccgc gcaggtctcg 60
tccagagcgc gatctcgctg ctcttcagcg cgatctgcgg ctcccgcggc gtgctccgcg 120
gggtctctca gggctggtac caggggcgtg ggggcggggc tgagcgagtc cagcccaggg 180
ctccggggac cgtttgtagt taggatccgc tgtggcgtcc tgagtggagt ttgggacccc 240
agggagggag ggtgtgggcg ttcgggtcca gaggagctgt ttaggtgcgt gtggaaggga 300
gctgcatgtg gcggtttttc tctgggccgg cggctctgag ggggagcgtg tgcgtgggtg 360
ggagggggct ggctggccct gcccctcaga gcgaccggag tcgccttatc ccctgggttg 420
gtgcggcccg aggctgaggg cgcggcgggg gatggggggg atgcggtgtt gcagcctcgg 480
gcgaggggaa gccaggacag gaggctgtga gggtgggagt cggccggcga actcccactg 540
cgggaggcgc acgggctgtg gttcctgggc tctgaggggc acggtagaca ggaaccccgc 600
ctcaggcgta tgccgtaatg cgaattggct gttctgtgaa aacctccaat ttttttattc 660
ttcgtggtaa attcgtaaat gcgaaatcgc actgtttctc taggtttacc cgggtgttgg 720
ggaagggctt tcgttagaat tttgctacac gtggttgtac acacgccacc aagggacata 780
ttcagagcgt ttactttaaa gggcatatcc tgtggagaaa tctgggtgtc cgtgaaccct 840
ggaagatccg gatgcttgtt gcaatcccac gcataagcgg ttatgcgggt gcttcccacc 900
ccatccccaa aacttcaagt cgtgccccga ctcaacaacg atttctgaag cgctcgttgg 960
gcttggcccg 970

<210> 4
<211> 970
<212> DNA/RNA
<213> Artificial Sequence

<220>
<221> misc_feature
<222> (1)..(970)
<223> y is c or u

<400> 4
yggggutta tuygyguttu uauuygygaa gaaggagygg ttguaguygy guaggutuyg 60
tuuagagygy gatutygutg ututtuagyg ygatutgygg utuuygyggy gtgutuygyg 120
gggtututua gggutggtau uaggggygtg ggggyggggu tgagygagtu uaguuuaggg 180
utuyggggau ygtttgtagt taggatuygu tgtggygtuu tgagtggagt ttgggauuuu 240
agggagggag ggtgtgggyg ttygggtuua gaggagutgt ttaggtgygt gtggaaggga 300
gutguatgtg gyggtttttu tutggguygg yggututgag ggggagygtg tgygtgggtg 360
ggagggggut ggutgguuut guuuutuaga gygauyggag tyguuttatu uuutgggttg 420
gtgygguuyg aggutgaggg ygyggygggg gatggggggg atgyggtgtt guaguutygg 480
gygaggggaa guuaggauag gaggutgtga gggtgggagt ygguyggyga autuuuautg 540
ygggaggygu aygggutgtg gttuutgggu tutgaggggu ayggtagaua ggaauuuygu 600
utuaggygta tguygtaatg ygaattggut gttutgtgaa aauutuuaat ttttttattu 660
ttygtggtaa attygtaaat gygaaatygu autgtttutu taggtttauu ygggtgttgg 720
ggaagggutt tygttagaat tttgutauay gtggttgtau auayguuauu aagggauata 780
ttuagagygt ttautttaaa ggguatatuu tgtggagaaa tutgggtgtu ygtgaauuut 840
ggaagatuyg gatguttgtt guaatuuuay guataagygg ttatgygggt guttuuuauu 900
uuatuuuuaa aauttuaagt ygtguuuyga utuaauaayg atttutgaag ygutygttgg 960
guttgguuyg 970

<210> 5
<211> 25

<212>  DNA
<213>  Primer

<400>  5
tgagtttatg gygatatttt tagtt                                          25

<210>  6
<211>  25
<212>  DNA
<213>  Primer

<400>  6
ctataattcc taaactctaa aaaac                                          25

<210>  7
<211>  25
<212>  DNA
<213>  Primer

<400>  7
   gttttttaga gtttaggaat tatag                                          25

<210>  8
<211>  24
<212>  DNA
<213>  Primer

<400>  8
caacacccac ttctataatt ttac                                           24

<210>  9
<211>  36
<212>  DNA
<213>  Primer

<400>  9
gaaagttttt ttttaatatt ygggtaaatt tagaga                              36

<210>  10
<211>  33
<212>  DNA
<213>  Primer

<400>  10
ctattctata aaaacctcca atttttttat tct                                 33

<210>  11
<211>  27
<212>  DNA
<213>  Primer

<400>  11
tattygggta aatttagaga aatagtg                                        27

<210>  12
<211>  28
<212>  DNA
<213>  Primer

<400>  12
ygatttygta tttaygaatt tattayga                                       28

## Claims

1. An isolated polynucleotide, wherein the isolated polynucleotide comprises:
(a) a polynucleotide with a nucleotide sequence as shown in SEQ ID NO: 1;
(b) a fragment of the polynucleotide of (a), having at least one CpG site with modification; and/or
(c) a nucleic acid complementary to the polynucleotide or fragment of (a) or (b).

2. The isolated polynucleotide according to claim 1, wherein the modification comprises 5-methylation, 5-hydroxymethylation, 5-formylcytosine or 5-carboxylcytosine.

3. The isolated polynucleotide according to claim 1, wherein, for (b), the fragment of the polynucleotide contains:
bases at positions 281 to 309 or positions 282 to 308 of SEQ ID NO: 1;
bases at positions 1 to 389 of SEQ ID NO: 1; or
bases at positions 418 to 970 of SEQ ID NO: 1.

4. An isolated polynucleotide, wherein, the polynucleotide is converted from the polynucleotide according to claim 1 or 2 or 3, and as compared with the sequence in claim 1 or 3, the cytosine C of the CpG site(s) with modification is unchanged, and the unmodified cytosine is converted into T or U.

5. The polynucleotide according to claim 4, wherein the polynucleotide comprises:
(d) a polynucleotide with a nucleotide sequence as shown in SEQ ID NO: 2 or 4;
(e) a fragment of the polynucleotide of (d), having at least one CpG site with modification.

6. The polynucleotide according to claim 5, wherein, for (e), the fragment of the polynucleotide contains:
bases at positions 281 to 309 or positions 282 to 308 of SEQ ID NO: 2;
bases at positions 1 to 389 of SEQ ID NO: 2;
bases at positions 418 to 970 of SEQ ID NO: 2;
bases at positions 662 to 690 or positions 663 to 689 of SEQ ID NO: 4;
bases at positions 582 to 970 of SEQ ID NO: 4; or
bases at positions 1 to 553 of SEQ ID NO: 4.

7. Use of the polynucleotide according to any of claims 1-6 in manufacture of a tumor detection agent or kit.

8. The use according to claim 7, wherein, the tumors comprise: hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; digestive system tumors such as esophageal cancer, gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

9. The use according to claim 7 or 8, wherein samples of the tumor comprise: tissue samples, paraffin embedded samples, blood samples, pleural effusion samples, and alveolar lavage fluid samples, ascites and lavage fluid samples, bile samples, stool samples, urine samples, saliva samples, sputum samples, cerebrospinal fluid samples, cell smear samples, cervical scraping or brushing samples, tissue and cell biopsy samples.

10. A method of preparing a tumor detection agent, comprising: providing the polynucleotide according to any of claims 1-6, designing a detection agent for specifically detecting a target sequence which is the full length or fragment of the polynucleotide; wherein, the target sequence has at least one modified CpG site; preferably, the detection agent comprises: primers or probes.

11. An agent or a combination of agents, wherein, the agent or the combination of agents specifically detect the modification on CPG site(s) of a target sequence, which is the full length or fragment of the polynucleotides according to any of claims 1-6 and has at least one modified CpG site.

12. The agent or the combination of agents according to claim 11, wherein the agent or combination of agents is for a gene sequence containing the target sequence, and the gene sequence comprises gene Panels or gene groups.

13. The agent or the combination of agents according to claim 11, wherein the agent or combination of agents comprise: primers or probes; preferably, the primers are:
primers shown in SEQ ID NO: 5 and 6;
primers shown in SEQ ID NO: 7 and 8; or
primers shown in SEQ ID NO: 9 and 10.

14. Use of the agent or combination of agents according to any of claims 11-13 in the manufacture of a kit for detecting tumors; preferably, the tumors comprise: hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; digestive system tumors such as esophageal cancer, gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

15. A detection kit, comprising:
container(s) and the agent or combination of agents according to any of claims 11-13 in the container(s).

16. A method of detecting the methylation profile of a sample *in vitro,* comprising:
(i) providing the sample and extracting nucleic acid;
(ii) detecting modification on CPG site(s) of a target sequence in the nucleic acid of (i), wherein the target sequence is the polynucleotide according to any of claims 1 to 2 or the polynucleotide converted therefrom according to any of claims 3 to 6.

17. The method according to claim 16, wherein, in step (3), the analysis methods comprise pyrosequencing, bisulfite conversion sequencing, method using methylation chip, qPCR, digital PCR, second generation sequencing, third generation sequencing, whole genome methylation sequencing, DNA enrichment detection, simplified bisulfite sequencing technology, HPLC, MassArray, methylation specific PCR, or their combination, as well as *in vitro* detection and *in vivo* tracer detection for the combined gene group of partial or all of the methylation sites in the sequence shown in SEQ ID NO: 1.

18. The method according to claim 16, wherein, step (ii) comprises:
(1) treating the product of (i) to convert unmodified cytosine into uracil; preferably, the modification includes 5-methylation, 5-hydroxymethylation, 5-formylcytosine or 5-carboxylcytosine; preferably, treating the nucleic acid of step (i) with bisulfite;
(2) analyzing the modification of the target sequence in the nucleic acid treated by (1).
